(19) Europäisches Patentamt
European Patent Office
Office européen des brevets

(11) EP 1 276 536 B1

## (12) EUROPEAN PATENT SPECIFICATION

(45) Date of publication and mention
of the grant of the patent:
**15.09.2004 Bulletin 2004/38**

(51) Int Cl.[7]: **A61N 1/04**, B65D 81/20

(21) Application number: **01927638.5**

(86) International application number:
**PCT/DK2001/000278**

(22) Date of filing: **26.04.2001**

(87) International publication number:
**WO 2001/080943 (01.11.2001 Gazette 2001/44)**

(54) **A METHOD FOR REDUCING DECOMPOSITION DURING STORAGE OF A SKIN ELECTRODE**

VERFAHREN ZUR REDUKTION DER ZERSETZUNG EINER HAUTELEKTRODE WÄHREND DER LAGERUNG

PROCEDE PERMETTANT DE REDUIRE LA DECOMPOSITION D'UNE ELECTRODE CUTANEE PENDANT L'ENTREPOSAGE

(84) Designated Contracting States:
**AT BE CH CY DE DK ES FI FR GB GR IE IT LI LU MC NL PT SE TR**

(30) Priority: **27.04.2000 DK 200000693**

(43) Date of publication of application:
**22.01.2003 Bulletin 2003/04**

(73) Proprietor: **MEDICOTEST A/S**
**3650 Olstykke (DK)**

(72) Inventors:
• **NIELSEN, Brian**
**DK-2100 Copenhagen (DK)**

• **THOMSEN, Steen**
**DK-2500 Valby (DK)**

(74) Representative: **Olsen, Lau Lund et al**
**Internationalt Patent-Bureau A/S,**
**Hoeje Taastrup Boulevard 23**
**2630 Taastrup (DK)**

(56) References cited:
**WO-A1-98/41278**       **DE-A1- 4 426 760**
**US-A- 4 674 512**       **US-A- 4 895 169**
**US-A- 5 960 708**

EP 1 276 536 B1

**Description**

TECHNICAL FIELD

The present invention relates to a method for reducing decomposition during storage of an electrode for establishing electrical contact with the skin. More specifically, the invention relates to a method for reducing decomposition during storage of physiological electrodes by means of which a plurality of physiological functions may be monitored or stimulated. Furthermore, the invention relates to a packaging method and a container comprising an electrode.

BACKGROUND ART

Electrodes establishing electrical contact with the skin are used for the administration of electrical signals to the body as well as collecting electrical signals generated in the body.

Electrical signals may be administered to the body of a patient through skin electrodes for a variety of reasons, including the treatment of fibrillation by administering an electric shock, treatment of pain and promotion of healing. The electric shock counteracts atrial or ventricular fibrillation of the heart and, if the treatment is successful, makes the rhythm of the heart revert to the normal mode.

Electric signals generated in the body may be collected by skin electrodes and monitored on a suitable monitoring device. In particular, the electrical signals of the heart may be monitored as an electrocardiogram (in the following abbreviated as ECG) to monitor the operation of the heart.

Skin electrodes have to meet a plurality of requirements to be suitable for supplying or measuring electrical signals, e.g. the skin electrodes must be sufficiently flexible to conform with the patient's body to secure a sufficient contact area, and to display satisfactory adhesion and electrical contact with the patient's body when the electrodes are placed properly. Also, a low impedance to ensure a good transmission of electric energy and a low polarization of the electrode is of importance. A special requirement for an electrode is that suitably it should be able to withstand storage without fast deterioration. Prolonged storage is valuable for a variety of reasons. For example, electrodes are frequently a part of the standard emergency equipment used by rescue teams and in remote areas. Therefore, the reliability of the electrodes may be crucial for saving lives. Furthermore, in the absence of a stabile and reliable electrode, the emergency equipment should constantly be surveyed and old not used electrodes must be disposed of.

It is well-known that electrodes may have a tendency to deteriorate during storage resulting in a reduced shelf life. In US 4,895,169 it is speculated that the reason for the deterioration is the presence of saline in the electrically conductive gel. To improve the shelf life it is suggested to use a tinstannous chloride electrode element. More specifically, it is suggested to use tin as the conductive metallic layer having affixed stannous chloride to the face of the layer pointing towards the electrically conductive gel.

In US 4,674,512 it is suggested to incorporate a stabilizer to prevent tin salt from reacting with other chemical constituents of the electrically conductive gel. The amount of the stabilizer is preferably sufficient for maintaining the tin ions in a solution within the electrically conductive gel. The stabilizer may be an acid group bearing compound, such as tartaric acid, a n-alkyl sulfonate, citrate, or sodium nitrate.

US 4,327,737 pertains to an electrode which comprises a metalized stud in contact with an electrically conductive gel. To impede evaporation of the moisture contained in the gel, the electrode is stored between sheets of paper coated with a thermoplastic material.

At present, electrodes for establishing electrically contact with the skin are preferably stored in bags in the interim period between production of the electrodes and the use thereof. The atmosphere the electrodes are packed in is the normal surrounding air.

In one aspect the present invention aims at providing a method for improving the shelf life of electrodes which are suitable for establishing electrical contact with the skin. Especially, it is the purpose of a certain aspect of the invention to provide a method for improving the shelf life of a physiological electrode comprising an electrically conductive gel which during storage is corrosive towards the used metal for the electrically conductive metallic layer.

DISCLOSURE OF THE INVENTION

The invention provides a method for reducing corrosion of an electrically conductive metallic layer during storage of an electrode comprising said electrically conductive metallic layer in intimate contact with an electrically conductive gel, said electrode being adapted for establishing electrical contact with the skin, wherein the access to oxygen is reduced or eliminated by storing said electrode in a suitable atmosphere.

The access to oxygen must be reduced or eliminated in at least a part of the storage time. Preferably, the access to oxygen is reduced or eliminated throughout the entire storage time.

As used herein the term "corrosion" refers to a substantial corrosion of the electrically conductive metallic layer reducing the total shelf life or the functional reliability of the electrode.

Reduction of the amount of oxygen may be obtained by storing the electrode in vacuum or in an atmosphere having a reduced concentration of oxygen compared to the concentration of oxygen in the surrounding air. Optionally, the packaging in vacuum and in an atmosphere having a reduced concentration of oxygen is combined.

In the event the electrode is stored in vacuum, the pressure is suitably within the range of 500 to 50000 Pa, preferably 5000 to 30000 Pa. In a preferred embodiment a bag of a gas-tight material is used for storing the electrode. Suitably a chamber having the desired low pressure (vacuum) is used for packaging the electrode. The electrode is then provided in the gas-tight bag of the chamber and the open edge(s) of the bag is(are) closed. The term "gas-tight" material is used herein in the sense that the permeation of gas through the material is substantially impeded, realizing that a material being absolute impermeable to gases hardly exists.

More than one electrode may be provided in each bag. As an example, two electrodes for simultaneous use may be contained in one bag.

The access to oxygen may also be reduced in a way that secures that the electrode, in particular the part of the electrode comprising the electrically conductive gel and the electrically conductive metallic layer, is contacted with a concentration of oxygen substantially below the normal level of oxygen in the atmosphere, i. e. below around 21 % by volume.

In the event the electrode is stored in an atmosphere having a reduced concentration of oxygen, the electrode may be stored in a room having the desired composition of atmosphere. More than one electrode may be stored in the same room. Preferably, however, the electrode is stored in a bag of gas-tight material. In a preferred method, a chamber having a desired composition of gas components is provided, wherein the concentration of oxygen is substantially below the concentration of oxygen in the surrounding air. In said chamber the electrode is packed in the gas-tight bag and the edge(s) closed, which secures that the gas in contact with the electrode during storage is deprived in oxygen.

In the event that a combination of storage in vacuum and in an atmosphere having a reduced concentration of oxygen, is used, the electrode may be packed in a chamber having a pressure below the normal level and a composition of atmosphere which is deprived of oxygen compared to the surrounding air. Preferably, the chamber comprising the electrode is, in a first step, evacuated to a first relatively low pressure, such as 500 to 50000 Pa, preferably 5000 to 30000 Pa. In a second step, a gas deprived in oxygen is let into the chamber to obtain a second pressure higher that the first but, preferable, below the normal pressure (101 kPa).

The reduction or elimination of access to oxygen in the method according to the invention is in a preferred embodiment secured by storing in a substantially inert atmosphere. As used herein "inert" refers to an atmosphere which is not reactive towards the electrode or parts thereof. Any inert gases may be used in the inert atmosphere. Preferably, the inert atmosphere contains nitrogen or argon, or a combination thereof. Nitrogen is preferred due to the relatively low cost thereof.

In another embodiment of the invention the access to oxygen is reduced or eliminated by storing in a reducing atmosphere containing hydrogen gas.

The concentration of the inert or the reducing gas(es) in the atmosphere is selected so that an improvement of the shelf life of the electrode is secured. Suitably, the concentration of the inert or reducing gas (es) is 95 % by volume or above, preferably 99 % by volume or above.

The electrode may be stored in any facility which has the ability to reduce or eliminate the access to oxygen. E.g. the electrode may be stored in a room, a chamber, or a container such as a bag. Generally, the electrode is stored in a container having substantially gas-tight walls. In a preferred embodiment, the electrode is packed in a bag, the walls of which may be composed of any material or combination of materials which can impede to a substantially extent the permeation of gases from the inside of the bag to the outside as well as gases in the surrounding air to the inside of the bag.

A single material for the walls of the bag is generally not able to fulfil all the functions desired for the bag. Therefore, a laminate of several layers of material is generally used. Due to the properties of aluminium to impede permeation of oxygen, it is preferred to include a layer of aluminium or aluminium alloy in the laminate. Besides the layer of aluminium or aluminium alloy the laminate suitably comprises one or more films of a plastic material. The plastic material may be selected from the group consisting of low-density polyethylene, high-density polyethylene, polypropylene, and polyamide. Preferably, the layer of aluminium is provided with at least one layer of a plastic material on each side to avoid damage of the metallic layer.

Various electrically conductive gels well-known to the person skilled in the art may be used in the method according to the invention. Preferred gels are prepared of hydrophilic polymers. In the following, gels prepared of hydrophilic polymers will be termed hydrogels. Hydrogels comprise an amount of water, which increases the skin compatibility and lower the electrical resistance.

The hydrophilic polymer may for instance be selected from the group consisting of polyacrylate, polymethacrylate, polyacrylamide, poly(vinyl alcohol), poly(ethylene oxide), poly(ethylene imine), carboxymethylcellulose, methyl cellulose, poly(acryl amide sulphonic acid), polyacrylonitril, poly(vinyl-pyrrolidone), agar, dextran, dextrin, carrageenan, xanthan, and guar.

The electrically conductive gel is preferably a flexible stiff gel which maintains the integrity during storage and application. However, the electrically conductive gel may be in the form of a viscous paste or creme, if so desired.

The pH of the electrically conductive gel may have any suitable value, i.e. the gel may be acidic, neutral or alkaline. In a preferred embodiment of the present invention, the electrically conductive gel provides for an acidic or an alkaline corrosion of the metallic layer. The acid or alkaline electrically conductive gel, respectively, may be provided in any suitable way. In one embodiment a mineral or organic acid or base, that provides for the eventually obtained pH, is added to the gel during the preparation thereof. Examples of mineral or organic acids that may be used are hydrochloric acid, sulphuric acid, nitric acid, phosphorus acid, acetic acid, formic acid, benzoic acid, and sulfonic acid. Examples of mineral or organic alkaline substances that may be used are ammonia, potassium hydroxide, sodium hydroxide, calcium hydroxide, pyridine, and aniline. In another embodiment, which is explained in more detail below, the polymers of the hydrogel structure itself contains acid or alkaline groups. In a third embodiment of the invention, a combination of the two preceding embodiments is used, i.e. the gel contains a mineral or organic acid or base added during the preparation as well as polymers carrying acid or alkaline groups.

If the corrosion of the metallic layer is performed in an acidic environment, it is preferred that the polymer comprises acid groups, such as carboxylic, sulphonic or nitric groups. In acidic environments such groups will predominantly be anionic and may thus be capable of transferring a cation carrying a charge between the skin of the patient and the metallic layer. A preferred polymer is polyacrylate or polymethacrylate, or a co-polymer containing acrylic acid or methacrylic acid as one of its monomers.

A polyacrylate at low pH may contain a fairly large amount of water providing a sticky gel with an ability to penetrate the small pores of the skin. In a preferred embodiment of the method according to the invention the content of water in the hydrogel is above 50% by weight, more preferred above 70% by weight, when the pH of the gel is between 1 and 3. The satisfactory coupling between the gel and the surface of the skin of a patient results in a low skin impedance. Furthermore, the electrode adheres well to the skin of the patient and remains in position during operation even if tension is applied thereto. The satisfactory coupling also ensures a high energy transfer resulting in substantially no burning of the skin.

If the corrosion of the metallic layer is provided in an alkaline environment, it is preferred that the ionizable groups of the polymeric structure are basic groups, such as amine, imine, or amide groups. In alkaline environments, such groups will predominantly be cationic and may thus be capable of carrying a free anion in the gel.

Preferably, the gel provides an acidic corrosion. The chosen pH of the electrically conductive gel depends on the selected metallic layer and may be determined by the person skilled in the art through routine experiments.

The pH of the electrically conductive gel is preferably between 0 and 4, more preferred between 1 and 3. The selected pH is a trade-off between skin compatibility and sufficient corrosion of the metallic layer. Therefore, the preferred metals for the electrically conductive metallic layer is selected among metals having a high sensitivity to acid or base. Preferred metals include tin, aluminium, zinc, silver, and lead and any combination thereof. Tin is the most preferred metal for the metallic layer. The purity of the used metal is usually high. Preferably, the purity is 99% by weight or more. The thickness of the metallic layer is not of particular importance to the present invention. A thickness of 0.05 mm has proved to be useful.

To be electrically conductive the hydrogel contains electrolytes, which carries the electrical charges. However, the presence of electrolytes also increases the tendency of the electrically conductive gel to be aggressive to the electrically conductive metallic layer even though the pH of the hydrogel is close to the neutral area. This tendency is attenuated if the gel is acidic or alkaline.

Although some ions may be etched from the metallic layer and may serve to transfer an electrical charge from the metallic layer to the skin surface, it may be desired to add further ions to improve the conductivity of the gel. The ions may be added as an ionizable salt. In principle, any ions having the ability to move within the gel may be used. However, preferred ionizable salts are KCl, KBr, NaCl, AgCl or $SnCl_2$.

The invention is based on the finding that the degradation of an electrode is reduced when the access to oxygen during storage is reduced or eliminated.

Without the intention of limiting the invention to any specific theory, it is believed that the observed reduction in decomposition of an electrode for establishing electrical contact with the skin can be found in the fact that oxygen participates in the cathode reaction. In the following, the proposed method of function is illustrated for a metallic layer of tin. The anode reaction in the corrosion of tin is thought to follow the equation

$$Sn \rightarrow Sn^{2+} + 2e^- \qquad (I)$$

The cathode reaction is believed to follow the equation

$$\tfrac{1}{2}O_2 + 2e^- + H_2O \rightarrow 2OH^- \qquad (II)$$

Thus, the total reaction (I+II) is

$$Sn + \tfrac{1}{2}O_2 + H_2O \rightarrow Sn(OH)_2 \qquad (III)$$

In consequence, the stannous hydroxide is precipitated on the surface of the metallic layer or in the electrically conductive gel.

When the access to oxygen is reduced or eliminated, the cathode reaction will not proceed or only proceed to a limited extent, and soon the anode reaction will be halted or decreased due to the absence of an electron acceptor.

If the gel is acidic, two competing cathode reactions are believed to exist:

$$2H^+ + 2e^- \rightarrow H_2 \qquad (IV)$$

and

$$\tfrac{1}{2}O_2 + 2H^+ + 2e^- \rightarrow H_2O \qquad (V)$$

Which one of the reactions (IV) and (V) that prevail depends on the environment.

Thus, if reaction (IV) prevails, the total reaction (I+IV) is thought to be

$$Sn + 2H^+ - \rightarrow Sn^{2+} + H_2 \qquad (VI)$$

As it may be inferred from equation VI the progress may be reduced if hydrogen gas is present during storage in the vicinity of the electrode.

If reaction (V) prevails, the total reaction (I + V) is thought to be

$$Sn + 2H^+ + \tfrac{1}{2}O_2 \rightarrow Sn^{2+} + H_2O \qquad (VII)$$

According to both total reactions (VI and VII) the stannous ion is maintained in a solution and is not inactivated as a salt. However, a precipitation is nevertheless seen during storage in the present of oxygen. At present, the composition of the precipitation is not known. It is speculated that the stannous ions, oxygen and possible further compounds react to produce stannous oxide (SnO). This theory is supported by the fact that precipitation is not observed, or only observed to a limited extent, when the electrode is stored in the absence of oxygen.

In one aspect of the present invention it pertains to a method in which a electrically conductive gel is used, wherein the pH of the gel is chosen so as to provide for a corrosion of the electrically conductive metallic layer. Without intending to limit the scope of the invention to a specific explanation or theory, at present it is believed that the chemical attack of the metallic layer provides a diminished impedance at the interface between the metallic layer and the acidic gel. The chemical attack will result in the creation of pits in the surface of the metallic layer, thus increasing the surface area so that the electrical contact between the gel and the metallic layer is improved. It is also believed that the generation of a relatively high concentration of metallic ions at the interface contributes to the availability of current carriers when a current is impressed, resulting in a reduced tendency to build-up charge, i.e. to serve as a capacitor.

The present invention also pertains to a method for packaging an electrode comprising an electrically conductive metallic layer in intimate contact with an electrically conductive gel, said method comprises the steps of

arranging the electrode in a substantially air-tight container,

evacuating the container and/or providing in the container an atmosphere having a reduced concentration of oxygen compared to the concentration of oxygen in the surrounding air,

sealing the container, whereby the electrode is provided in a substantially air-tight closure deprived of oxygen.

The packaging method ensures that the electrode can be stored in an environment wherein the access to oxygen is reduced or eliminated, compared to the ambient air. The atmosphere provided in the container is preferably a substantially inert or reducing atmosphere. A preferred inert atmosphere is nitrogen or argon gas, preferably in a concentration of 99 % by volume or above.

Suitably, the container is a bag composed of a laminate comprising a layer of aluminium or aluminium alloy.

The evacuating step of the packaging method preferably comprises placing the container harboring the electrode in a vacuum chamber and evacuating the vacuum chamber to a pressure of 500 to 50000 Pa, preferably 5000 to 30000 Pa. The use of a vacuum chamber usually provides for an even distribution of pressure and injected gases in the container, thus preventing local envelopes of oxygen enriched areas. The evacuation step preferably further comprises injecting an inert or reducing gas in the container subsequent to the evacuation. The injection of the inert or reducing gas into the container provides for a rinsing effect. The injection provides for an effective substitution of the atmosphere inside the container with an atmosphere enriched with the injected inert or reducing gas.

The inert or reducing gas injected into the container is suitably dosed in an amount to increase the pressure to a level above the vacuum pressure but below ambient pressure.

The packaging method may be used for any electrode having an electrically conductive metallic layer in intimate contact with an electrically conductive gel. However, the reduction in the corrosion is in particular noted when the pH of the electrically conductive gel is between 0 and 4.

The present invention also pertains to a container for storage having walls of an essentially gas-tight material, comprising

(i) an electrode comprising an electrically conductive metallic layer in intimate contact with an electrically conductive gel and

(ii) an atmosphere having a reduced amount of oxygen compared to the amount of oxygen in the surrounding air.

Preferably, the atmosphere in step (ii) comprises of an essentially inert atmosphere. Alternatively, the atmosphere comprises an essentially reducing atmosphere of hydrogen. Suitably, the concentration of the inert or reducing gas(es) in the atmosphere is 95% by volume or above, preferably 99% by volume or above.

Besides the electrically conductive metallic layer and the electrically conductive gel the electrode suitably comprises further elements. Preferably, the face of the metallic layer opposite to the face attached to the electrically conductive gel has a suitable insulating cover in order to reduce the risk that the operator will get an electric shock too during use. The insulating cover is preferably prepared of a polymeric material such as polyolefine, e.g. polyethylene or polypropylene.

The metallic layer may be connected to instruments or devices that are utilized in connection with skin electrodes in any suitable way. It is, however, preferred that the electrically conductive metallic layer is connected with a point adapted to mate with a corresponding portion of a connector, because, especially in an emergency situation, the electrodes may easily be connected to the devices or the instrument. Furthermore, the presence of a point adapted to mate with the corresponding parts of a connector of an instrument or device allows the electrode to be a disposable article. Thus, the electrode may be adapted for single use and disposable in any suitable way.

Whereas the electrically conductive gel used in the present invention is preferably a hydrogel having the ability to adhere to the skin of the patient, it may be preferred to cover the face opposing the face in contact with the metallic layer by a second or further electrically conductive skin adhering layer(s) having a pH more compatible with the skin of the patient. The pH of the second gel layer is preferably 5-9.

During storage the surface of the electrically conductive layer or layers intended to adhere to the skin is provided with a liner. Immediately before use, the liner is removed.

The electrode according to the present invention may be used for a variety of applications, including. monitoring, stimulation, therapeutical and surgical applications.

The monitoring applications include any measurement of the condition of the muscles or nerves of the human or animal body. Specific examples for the use of the electrode according to the present invention for monitoring applications are ECG, EMG (electromyography) and EEG (electroencephalography).

The stimulating applications include any method for stimulation of the muscles or nerves of the human or animal body. Specific examples of the use of the electrode according to the present invention for stimulating applications are for defibrillation, pacing and pain relief.

Examples of therapeutical applications of the electrode according to the present invention are for electro therapy of muscles and nerves.

The electrode according to the present invention may also be used for surgical applications as grounding plate. A grounding plate is used in a special surgical technique wherein the tissue of the patient is cut with a needle supplied with a high voltage. When the needle supplied with the high voltage comes into contact with the skin heat will be developed and the tissue may be cut. The grounding plate is used to close the electrical circuit. To avoid burning, the grounding plate usually has a fairly large size.

EXAMPLES

Example 1

Preparation of a bag containing an electrode in vacuum.

First an electrode was prepared. A precursor for the electrically conductive gel was prepared by mixing 7,670 g water, 25 g KCl and 2,250 g acrylic acid during agitation. The agitation continued until all the KCl was dissolved. Subsequently, 24 g triethyleneglycol dimethacrylate and Darocure 1173 was added and the mixture was agitated in the dark for further 30 min. The precursor was then cast in a thickness of 1 mm in a matrix defined by a surrounding frame and a conductive metallic layer of tin. The precursor was subsequently irradiated by UV light to cure the precursor to a electrically conductive gel. Then, a release liner was attached to the surface of the gel and the frame. A backing was attached to the face of the tin layer opposite to the face covered with the gel. The pH value of the cured gel was 2.

This electrode was placed in a bag in a vacuum chamber. The bag was prepared of two rectangular layers of laminate welded together on three edges. The laminate itself, was prepared of a layer of aluminium covered on both sides with a layer of polyethylene. The forth edge of the bag was placed between two welding jaws. Subsequently, the vacuum chamber was evacuated with a pump to a pressure of 10 kPa and the welding jaws were pressed together to obtain a welded joint. Finally, the pressure in the chamber was allowed to rise to the normal level and the bag containing the electrode was removed from the chamber.

The bag comprising the electrode was placed in a room for storage at standard conditions. After three months the electrode was examined and tested. The electrode showed satisfactory electrical abilities comparable to freshly prepared electrodes. An examination of the individual layers showed that only small pits were created at the surface of the metallic layer in intimate contact with the electrically conductive gel.

Example 2

Preparation of a bag containing an electrode in an inert atmosphere.

An electrode as prepared in Example 1 was placed in a bag in a vacuum chamber. The bag was prepared of two rectangular layers of laminate welded together on three edges. The laminate itself, was prepared of a layer of aluminium covered on both sides with a layer of polyethylene. The forth edge of the bag was placed between two welding jaws. Subsequently, the vacuum chamber was evacuated to a pressure of 3 kPa and, next, nitrogen gas ($N_2$) was transferred to the chamber from a nitrogen pressure container until a pressure of 65 kPa was obtained in the chamber. The nitrogen was let to the chamber through a nozzle pointing towards the interior of the bag to secure a rinse of the atmosphere in the bag. Following the inlet of nitrogen, the welding jaws were pressed together to obtain a welded joint. Finally, the pressure in the chamber was allowed to rise to the normal level and the bag containing the electrode was removed from the chamber.

The bag comprising the electrode was placed in a room for storage at standard conditions. After three months the electrode was examined and tested. The electrode showed satisfactory electrical abilities comparable to freshly prepared electrodes. An examination of the individual layers showed that only small pits were created at the surface of the metallic layer in intimate contact with the electrically conductive gel.

Example 3

Preparation of a bag containing an electrode in an reducing atmosphere.

An electrode as prepared in Example 1 was placed in a bag in a vacuum chamber. The bag was prepared of two rectangular layers of laminate welded together on three edges. The laminate itself, was prepared of a layer of aluminium covered on both sides with a layer of polyethylene. The forth edge of the bag was placed between two welding jaws. Subsequently, the vacuum chamber was evacuated to a pressure of 3 kPa and, next, hydrogen gas ($H_2$) was transferred to the chamber from a hydrogen pressure container until a pressure of 65 kPa was obtained in the chamber. The hydrogen was let to the chamber through a nozzle pointing toward the interior of the bag to secure a rinse of the atmosphere in the bag. Following the inlet of hydrogen, the welding jaws were pressed together to obtain a welded joint. Finally, the pressure in the chamber was allowed to rise to the normal level and the bag containing the electrode was removed from the chamber.

The bag comprising the electrode was placed in a room for storage at standard conditions. After three months the electrode was examined and tested. The electrode showed satisfactory electrical abilities comparable to freshly prepared electrodes. An examination of the individual layers showed that only small pits were created at the surface of the metallic layer in intimate contact with the electrically conductive gel.

**Claims**

1.  A method for reducing corrosion of an electrically conductive metallic layer during storage of an electrode comprising said electrically conductive metallic layer in intimate contact with an electrically conductive gel, said electrode being adapted for establishing electrical contact with the skin, wherein the access to oxygen is reduced or eliminated by storing said electrode in a suitable atmosphere.

2.  The method according to claim 1, wherein the electrode is stored in vacuum.

3.  The method according to claim 1, wherein the access to oxygen is reduced by storing in an atmosphere having a reduced concentration of oxygen compared to the concentration of oxygen in the surrounding air.

4.  The method according to any of the claims 1 to 3, wherein the access to oxygen is reduced by storing in a substantially inert atmosphere.

5.  The method according to claim 4, wherein the atmosphere contains nitrogen or argon gas.

6.  The method according to any of the claims 1, 2 or 3, wherein the access to oxygen is reduced by storing in a reducing atmosphere containing hydrogen gas.

7.  The method according to any of the precedent claims, wherein the concentration of inert or reducing gas in the atmosphere is 99% by volume or above.

8.  The method according to any of the precedent claims, wherein the electrode is stored in a substantially gas-tight container.

9.  The method according to claim 8, wherein the gas-tight container is a bag composed of a laminate comprising a layer of aluminium or aluminium alloy.

10. The method according to clam 1, wherein the pH of the electrically conductive gel is between 0 and 4.

11. The method according to any of the preceding claims, wherein the electrically conductive metallic layer comprises tin, aluminium, zinc, lead, or silver.

12. A method for packaging an electrode comprising an electrically conductive metallic layer in intimate contact with an electrically conductive gel, said method comprises the steps of

arranging the electrode in a substantially air-tight container,

evacuating the container and/or providing in the container an atmosphere having a reduced concentration of oxygen compared to the concentration of oxygen in the surrounding air,

sealing the container, whereby the electrode is provided in a substantially air-tight closure deprived of oxygen.

13. The method according to claim 12, wherein the atmosphere provided in the container is a substantially inert atmosphere.

14. The method according to claim 13, wherein the inert atmosphere is nitrogen or argon gas.

15. The methods according to any of the claims 12 to 14, wherein the concentration of inert or reducing atmosphere provided in the container is 99 % by volume or above.

16. The method according to claim 12, wherein the container is a bag composed of a laminate comprising a layer of aluminium or aluminium alloy.

17. The method according to claim 12, wherein the evacuating step comprises placing the container harboring the electrode in a vacuum chamber and evacuating the vacuum chamber.

18. The method according to claim 17, wherein the evacuation step further comprises injecting an inert or reducing gas in the container subsequent to the evacuation.

19. The method according to claim 18, wherein the amount of inert or reducing gas injected into the container increases the pressure to a level above the vacuum but below ambient pressure.

20. The method according to any of the claims 12 to 19, wherein the pH of the electrically conductive gel is between 0 and 4.

21. A container for storage having walls of an essentially gas-tight material comprising

(i) an electrode comprising an electrically conductive metallic layer in intimate contact with an electrically conductive gel and
(ii) an atmosphere having a reduced amount of oxygen compared to the amount of oxygen in the surrounding air.

22. The container according to claim 21, wherein the atmosphere consists of a substantially inert atmosphere.

23. The container according to claim 21, wherein the atmosphere consists of a substantially reducing atmosphere of hydrogen.

**Patentansprüche**

1. Verfahren zur Verminderung der Korrosion einer elektrisch leitfähigen metallischen Schicht während Lagerung einer Elektrode, umfassend die elektrisch leitfähige metallische Schicht in innigem Kontakt mit einem elektrisch leitfähigen Gel, wobei die Elektrode zur Erzeugung eines elektrischen Kontakts mit der Haut eingerichtet ist, wobei der Zugang zu Sauerstoff durch Lagern der Elektrode in einer geeigneten Atmosphäre vermindert oder eliminiert ist.

2. Verfahren nach Anspruch 1, wobei die Elektrode im Vakuum gelagert wird.

3. Verfahren nach Anspruch 1, wobei der Zugang zu Sauerstoff durch Lagern in einer Atmosphäre mit einer verminderten Konzentration an Sauerstoff im Vergleich zu der Konzentration von Sauerstoff in der umgebenden Luft reduziert ist.

4. Verfahren nach einem der Ansprüche 1 bis 3, wobei der Zugang zu Sauerstoff durch Lagern in einer im wesentlichen inerten Atmosphäre vermindert ist.

5. Verfahren nach Anspruch 4, wobei die Atmosphäre Stickstoff- oder Argongas enthält.

6. Verfahren nach einem der Ansprüche 1, 2 oder 3, wobei der Zugang zu Sauerstoff durch Lagern in einer reduzierenden Atmosphäre, die Wasserstoffgas enthält, vermindert wird.

7. Verfahren nach einem der vorhergehenden Ansprüche, wobei die Konzentration an inertem oder reduzierendem Gas in der Atmosphäre 99 Vol.-% oder darüber beträgt.

8. Verfahren nach einem der vorhergehenden Ansprüche, wobei die Elektrode in einem im wesentlichen gasdichten Behälter gelagert wird.

9. Verfahren nach Anspruch 8, wobei der gasdichte Behälter ein Beutel, aufgebaut aus einem Laminat, umfassend eine Schicht von Aluminium oder Aluminiumlegierung, ist.

**10.** Verfahren nach Anspruch 1, wobei der pH des elektrisch leitfähigen Gels zwischen 0 und 4 liegt.

**11.** Verfahren nach einem der vorhergehenden Ansprüche, wobei die elektrisch leitfähige metallische Schicht Zinn, Aluminium, Zink, Blei oder Silber umfaßt.

**12.** Verfahren zum Einpacken einer Elektrode, umfassend eine elektrisch leitfähige metallische Schicht in innigem Kontakt mit einem elektrisch leitfähigen Gel, wobei das Verfahren die Schritte des Anordnens der Elektrode in einem im wesentlichen luftdichten Behälter, des Evakuierens des Behälters und/oder des Vorsehens einer Atmosphäre mit einer verminderten Konzentration an Sauerstoff im Vergleich zu der Konzentration von Sauerstoff in der umgebenden Luft in dem Behälter und des Verschließens des Behälters umfaßt, wobei die Elektrode in einem im wesentlichen luftdichten Verschluß, wobei Sauerstoff entzogen ist, angeordnet ist.

**13.** Verfahren nach Anspruch 12, wobei die in dem Behälter vorgesehene Atmosphäre eine im wesentlichen inerte Atmosphäre ist.

**14.** Verfahren nach Anspruch 13, wobei die inerte Atmosphäre Stickstoff- oder Argongas ist.

**15.** Verfahren gemäß einem der Ansprüche 12 bis 14, wobei die Konzentration an inerter oder reduzierender Atmosphäre, die in dem Behälter vorliegt, 99 Vol.-% oder darüber beträgt.

**16.** Verfahren nach Anspruch 12, wobei der Behälter ein Beutel, aufgebaut aus einem Laminat, umfassend eine Schicht von Aluminium oder Aluminiumlegierung, ist.

**17.** Verfahren nach Anspruch 12, wobei der Evakuierungsschritt das Stellen des Behälters, welcher die Elektrode beherbergt, in eine Vakuumkammer und das Evakuieren der Vakuumkammer umfaßt.

**18.** Verfahren nach Anspruch 17, wobei der Evakuierungsschritt weiter das Injizieren eines inerten oder reduzierenden Gases in den Behälter nachfolgend der Evakuierung umfaßt.

**19.** Verfahren nach Anspruch 18, wobei die Menge an inertem oder reduzierendem Gas, welches in den Behälter injiziert wird, den Druck auf einen Grad oberhalb des Vakuums, aber unterhalb des Umgebungsdrucks erhöht.

**20.** Verfahren nach einem der Ansprüche 12 bis 19, wobei der pH des elektrisch leitfähigen Gels zwischen 0 und 4 liegt.

**21.** Behälter zur Lagerung mit Wänden aus einem im wesentlichen gasdichten Material, umfassend

(i) eine Elektrode, umfassend eine elektrisch leitfähige metallische Schicht in innigem Kontakt mit einem elektrisch leitfähigen Gel, und
(ii) eine Atmosphäre mit einer verminderten Menge an Sauerstoff, verglichen zu der Menge an Sauerstoff in der umgebenden Luft.

**22.** Behälter nach Anspruch 21, wobei die Atmosphäre aus einer im wesentlichen inerten Atmosphäre besteht.

**23.** Behälter nach Anspruch 21, wobei die Atmosphäre aus einer im wesentlichen reduzierenden Atmosphäre von Wasserstoff besteht.

**Revendications**

**1.** Procédé permettant de réduire la corrosion d'une couche métallique électriquement conductrice durant l'entreposage d'une électrode comprenant ladite couche métallique électriquement conductrice en contact intime avec un gel électriquement conducteur, ladite électrode étant adaptée à l'établissement d'un contact électrique avec la peau, dans lequel l'accès à l'oxygène est réduit ou éliminé en entreposant ladite électrode dans une atmosphère appropriée.

**2.** Le procédé selon la revendication 1, dans lequel l'électrode est entreposée sous vide.

**3.** Le procédé selon la revendication 1, dans lequel l'accès à l'oxygène est réduit par entreposage dans une atmosphère ayant une concentration en oxygène réduite comparée à la concentration en oxygène dans l'air environnant.

**4.** Le procédé selon l'une quelconque des revendications 1 à 3, dans lequel l'accès à l'oxygène est réduit par entreposage dans une atmosphère substantiellement inerte.

**5.** Le procédé selon la revendication 4, dans lequel l'atmosphère contient du gaz azote ou argon.

**6.** Le procédé selon l'une quelconque des revendications 1, 2 ou 3, dans lequel l'accès à l'oxygène est réduit par entreposage dans une atmosphère réductrice contenant du gaz hydrogène.

**7.** Le procédé selon l'une quelconque des revendications précédentes, dans lequel la concentration du gaz inerte ou réducteur dans l'atmosphère est de 99 % en volume ou plus.

8. Le procédé selon l'une quelconque des revendications précédentes, dans lequel l'électrode est entreposée dans un récipient substantiellement étanche aux gaz.

9. Le procédé selon la revendication 8, dans lequel le récipient étanche aux gaz est un sac composé d'un laminé comprenant une couche d'aluminium ou d'alliage d'aluminium.

10. Le procédé selon la revendication 1, dans lequel le pH du gel électriquement conducteur est entre 0 et 4.

11. Le procédé selon l'une quelconque des revendications précédentes, dans lequel la couche métallique électriquement conductrice comprend de l'étain, de l'aluminium, du zinc, du plomb ou de l'argent.

12. Procédé d'emballage d'une électrode comprenant une couche métallique électriquement conductrice en contact intime avec un gel électriquement conducteur, ledit procédé comprend les étapes suivantes :

disposer l'électrode dans un récipient substantiellement étanche à l'air,
mettre sous vide le récipient et/ou fournir dans le récipient une atmosphère ayant une concentration en oxygène réduite comparée à la concentration en oxygène dans l'air environnant, sceller le récipient, au moyen de quoi l'électrode est fournie dans une enceinte substantiellement étanche à l'air privée d'oxygène.

13. Le procédé selon la revendication 12, dans lequel l'atmosphère fournie dans le récipient est une atmosphère substantiellement inerte.

14. Le procédé selon la revendication 13, dans lequel l'atmosphère inerte est un gaz azote ou argon.

15. Les procédés selon l'une quelconque des revendications 12 à 14, dans lesquels la concentration de l'atmosphère inerte ou réductrice fournie dans le récipient est de 99 % en volume ou plus.

16. Le procédé selon la revendication 12, dans lequel le récipient est un sac composé d'un laminé comprenant une couche d'aluminium ou d'alliage d'aluminium.

17. Le procédé selon la revendication 12, dans lequel l'étape de mise sous vide comprend le fait de placer le récipient abritant l'électrode dans une chambre à vide et de mettre sous vide la chambre à vide.

18. Le procédé selon la revendication 17, dans lequel l'étape de mise sous vide comprend de plus l'injection d'un gaz inerte ou réducteur dans le récipient subséquemment à la mise sous vide.

19. Le procédé selon la revendication 18, dans lequel la quantité de gaz inerte ou réducteur injectée dans le récipient augmente la pression jusqu'à un niveau supérieur au vide mais inférieur à la pression ambiante.

20. Le procédé selon l'une quelconque des revendications 12 à 19, dans lequel le pH du gel électriquement conducteur est entre 0 et 4.

21. Récipient pour l'entreposage ayant des parois d'un matériau essentiellement étanche aux gaz comprenant :

(i) une électrode comprenant une couche métallique électriquement conductrice en contact intime avec un gel électriquement conducteur et
(ii) une atmosphère ayant une quantité réduite d'oxygène comparée à la quantité d'oxygène dans l'air environnant.

22. Le récipient selon la revendication 21, dans lequel l'atmosphère consiste en une atmosphère substantiellement inerte.

23. Le récipient selon la revendication 21, dans lequel l'atmosphère consiste en une atmosphère substantiellement réductrice d'hydrogène.